# EUROPEAN PATENT APPLICATION

(11) **EP 2 223 678 A1**
(43) Date of publication of application: **01.09.2010**
(21) Application number: 08866689.6
(22) Date of filing: 25.12.2008
(51) Int. Cl.: A61K 8/25, A61K 8/26, A61K 8/31, A61K 8/92, A61Q 1/08, A61Q 1/10, A61Q 1/12

(54) **METHOD FOR PRODUCING A COSMETIC PREPARATION**

(30) Priority: 28.12.2007 JP 2007339995; 28.12.2007 JP 2007339996
(71) Applicant: Kao Corporation, Chuo-Ku Tokyo 103-8210 (JP)
(72) Inventor: IMAI, Takamitsu, Tokyo 131-8501 (JP); FUKUDA, Ikuo, Tokyo 131-8501 (JP)
(74) Representative: HOFFMANN EITLE
(86) International application number: PCT/JP2008/003967
(87) International publication number: WO 2009/084205

(57) **Abstract**

Provided is a method of producing a cosmetic by molding a powder composition under pressure, the method including: applying a vibration having a frequency of 10 to 40 kHz and an amplitude of 10 to 100 µm to a powder composition while the powder composition is molded under pressure, the powder composition containing: (A) a powder having an average particle size of 0.1 to 300 µm; (B) an oil which is liquid at 25°C; and (C) an oil which is solid at 25°C, in which: the content of the component (A) is 75 to 95% by mass; and a mass ratio of the component (B) to the component (C) " (B) : (C) is 6:4 to 9.5:0.5.

## Description

### Field of the Invention

The present invention relates to a method for producing a cosmetic (cosmetic pressed powder) obtained by compressing a powder.

### Background of the Invention

A cosmetic obtained by compressing a powder is produced by mixing an oily component or the like into a powder and then milling and compressing the mixture. Some studies have been conducted to improve its qualities in use, such as attachment thereof to an applicator, attachment to the skin, and moisture retention. In addition, some other studies on compressing methods have also been conducted to improve stability such as shape retention and impact resistance. Further, in recent years, a make-up cosmetic such as an eye shadow, a blusher, or a foundation having excellent glossiness of appearance and glossiness of an applied film are strongly desired.

It has been known that, when a powder composition is molded, molding under pressure with ultrasonic vibration being appliedprovides a moldedproduct with an excellent impact resistance and a uniform powder removal. For example, Patent Document 1 describes that, when a powder cosmetic is molded while an ultrasonic wave is applied, air taken into a powder composition is taken out in a smooth and favorable manner, and as a result, a uniform, dense powder cosmetic is provided. Besides, Patent Document 2 describes that, after a container is filled with a composition containing an organic powder having an average compressive ultimate strength of 0.05 to 1 kg/mm², the composition is subjected to molding under pressure at a pressure of not more than 20 kg/cm² while vibration is being applied, and as a result, there is provided, without the crushing of the soft organic powder, a uniformly-molded product which is excellent in impact resistance and good in powder removal. In addition, Patent Document 3 describes that a powder mixture containing a thermoplastic product at 5 to 80 wt% is applied with ultrasonic force while being under pressure, providing a product which is partially breakable.

However, each of the powder cosmetics obtained by those methods was not good in glossiness on the surface of the molded cosmetic, and was not sufficiently satisfactory in terms of quality in use and stability.
[Patent Document 1] JP-A-63-275511
[Patent Document 2] JP-A-2003-261418
[Patent Document 3] JP-A-5-70325

### Summary of the Invention

### Problems to be solved by the Invention

The present invention relates to a method for producing a cosmetic which is excellent in terms of glossiness on the surface of a molded powder cosmetic, quality in use, glossiness of an applied film, and stability.

### [Means for solving the Problems]

The inventors of the present invention have found that, by using a combination of a powder, a liquid oil, and a solid oil at a specific ratio and subj ecting the combination to molding under pressure while irradiating the combination with an ultrasonic wave, the orientation of the powder is enhanced, and as a result, provided is a cosmetic which is uniformly attached to an applicator, and is excellent in terms of quality in use such as attachment (adhesion) to the skin and moisture retention, and stability such as shape retention and impact resistance, and which further is excellent in terms of glossiness on the surface of the molded cosmetic.

The present invention provides a method of compressing a cosmetic, the method including applying a vibration having a frequency of 10 to 40 kHz and an amplitude of 10 to 100 µm to a powder composition while the powder composition is molded, the powder composition containing the following components (A) to (C):
(A) a powder having an average particle size of 0.1 to 300 µm;
(B) an oil which is liquid at 25°C; and
(C) an oil which is solid at 25°C, in which the content of the component (A) is 75 to 95% by weight and a mass ratio of the component (B) to the component (C) "(B):(C)" is 6:4 to 9.5:0.5.
In addition, the present invention provides a cosmetic obtained by the method of compressing a cosmetic.

### Effect of the Invention

According to the present invention, there canbeprovided a cosmetic which is excellent in terms of glossiness on the surface of the molded cosmetic, glossiness of an applied film, quality in use and stability, and which can be finely and uniformly attached to an applicator, can be uniformly attached to the skin, and can offer moisture retention in application, and moreover, which is excellent in terms of impact resistance and shape retention, for example, when the cosmetic is transported or dropped.

### [Brief Description of the Drawing]

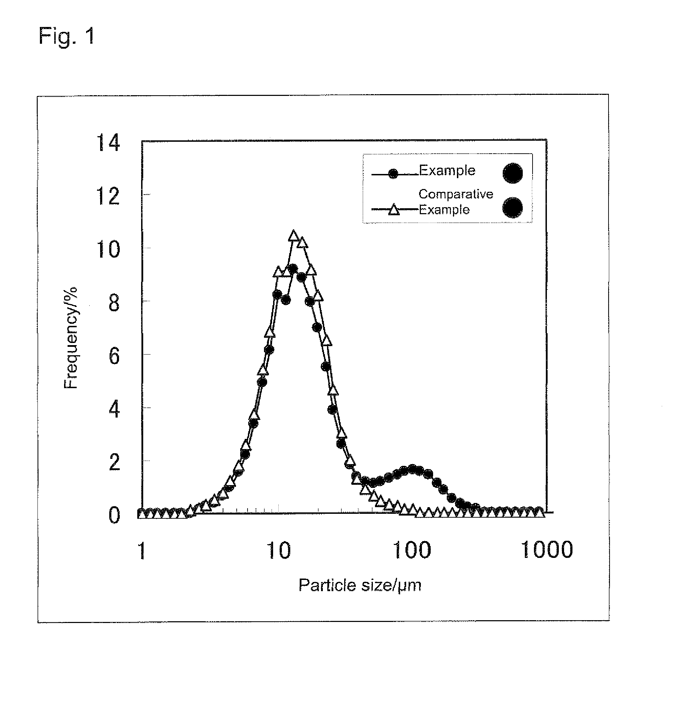

[FIG. 1] FIG. 1 is a graph illustrating a particle size distribution of all powder components contained in each of the powder cosmetics obtained in Example 28 and Comparative Example 7.

### Detailed Description of the Invention

A powder as the component (A) used in the present invention refers to a powder which is used in an ordinary cosmetic and which is insoluble in water and an oil, and may be any of a spherical, plate-like, and amorphous powders. When a plate-like powder is contained, glossiness on the surface of a molded cosmetic can be additionally enhanced. Examples of the powder may include: an inorganic powder such as silicic anhydride, magnesium silicate, talc, sericite, mica, synthetic mica, kaolin, boron nitride, flake glass, aluminum, colcothar, clay, bentonite, bismuth oxychloride, zirconium oxide, magnesium oxide, zinc oxide, aluminum oxide, calcium sulfate, barium sulfate, magnesium sulfate, calcium carbonate, magnesium carbonate, titanium oxide, iron oxide, ultramarine blue, chromium oxide, chromium hydroxide, carmine, carbon black, or a complex thereof ; an organic powder such as polyamide, polyester, polypropylene, polystyrene, polyurethane, a vinyl resin, a urea resin, a phenol resin, a silicone resin, an acrylic resin, a melamine resin, an epoxy resin, a polycarbonate resin, a divinylbenzene-styrene copolymer, a silk powder, cellulose, CI pigment yellow, CI pigment orange, a metal salt of a long-chain alkyl phosphoric acid, a metal salt of a higher fatty acid, an N-mono long-chain alkyl acyl basic amino acid, an N-mono long-chain alkyl acyl basic amino acid metal salt, a silicone resin, or a complex thereof; and a complex of the aforementioned inorganic powder and organic powder.

Of those, the plate-like powder is, for example, silicic anhydride, magnesiumsilicate, talc, sericite, mica, synthetic mica, kaolin, boron nitride, flake glass, aluminum, bismuth oxychloride, zinc oxide, aluminum oxide, calcium sulfate, barium sulfate, calcium carbonate, titanium oxide, iron oxide, a metal salt of a long-chain alkyl phosphoric acid, an N-mono long-chain alkyl acyl basic amino acid, or an N-mono long-chain alkyl acyl basic amino acid metal salt.

Further, a pearl pigment may be given as the plate-like powder. Examples of the pearl pigment include pigments each containing a thin base material and a coating layer in which the base material is selected from, for example, mica, synthetic mica, silica, glass, and aluminum, and the coating layer is selected from a metal, a metal oxide, a metal complex, and an organic pigment. Specific examples thereof include mica coated with titanium oxide, mica coated with iron oxide, glass coated with titanium oxide, and mica coated with iron oxide and titanium oxide.

Of those, preferred are flake glass and a pearl pigment, and more preferred is a plate-like powder such as flake glass (pearl pigment) coated with a metal oxide because of being excellent in glossiness and transparency on the surface of the molded cosmetic.

In addition, the plate-like powder has an aspect ratio of preferably 20 to 400, or more preferably 30 to 400. Containing the plate-like powder described above provides a cosmetic which is excellent in terms of moisture retention in application, glossiness and transparency on the surface of the molded cosmetic, and moreover, provides a cosmetic which offers a beautiful cosmetic-applied film having high glossiness.

The powder as the component (A) has an average particle size of 0.1 to 300 µm, or preferably 1 to 100 µm. It should be noted that the average particle size refers to a value of a median size obtained by dispersing the powder in ethanol and measuring the size using a laser diffraction particle size analyzer (LA-920, HORIBA, Ltd.).
Meanwhile, the aspect ratio refers to a value obtained by dividing the average particle size by the average thickness of particles. The average thickness of particles refers to a value obtained as described below. That is, the thicknesses of particles are measured using an electron microscope, and the total value of the thicknesses is divided by the number of the particles measured.

The powder as the component (A) may also be subjected to a surface treatment before use. Examples of the surface treatment may include a silicone treatment, a fatty acid treatment, an amino acid treatment, a lecithin treatment, a metal soap treatment, an alkyl treatment, a fluorine compound treatment, an ester treatment, and combined use of these treatments.
Specifically, the silicone treatment may be a treatment with methyl hydrogen polysiloxane, methyl polysiloxane, trimethyl siloxysilicate, a silicone resin, or the like, the fatty acid treatment may be a treatment with myristic acid, stearic acid, or the like, and the fluorine compound treatment may be a treatment with perfluoroalkyl phosphate, perfluoroalkylsilane, or the like.

The amount of a surface-treating agent used is preferably 0.1 to 10% by mass, or more preferably 2 to 9% by mass with respect to the weight of a powder before the treatment. A method for the surface treatment is not particularly limited, and the treatment can be carried out according to an ordinary method.

One or more kinds of the powder as the component (A) may be used, and are contained in the total composition at 75 to 95% by mass, preferably 85 to 95% by mass, or more preferably 85 to 92% by mass. If the content is in the above-mentioned range, attachment to an applicator is fine and uniform, attachment to the skin is uniform, and moisture retention is provided when the cosmetic is applied.

Further, the powder as the component (A) contains one or more kinds of plate-like powders selected from mica, synthetic mica, sericite, and a pearl pigment, in total, preferably at 30 to 95% by mass, or more preferably at 50 to 80% by mass, in terms of providing glossiness on the surface of the molded cosmetic and moisture retention in application.

An oil which is a liquid at 25°C (liquid oil) as the component (B) can be used without any limitation as long as it is used for common cosmetics. Examples thereof include: oils and fats such as cacao butter, castor oil, jojoba oil, olive oil, sunflower oil, and macadamia nut oil; higher fatty acids such as isononanoic acid and isostearic acid; fatty acid esters such as isopropyl myristate, isopropyl isostearate, neopentyl glycol dicaprate, and diisostearyl malate; hydrocarbon oils such as liquid paraffin, liquid isoparaffin, and squalane; silicone oil; and fluorine oil.

One or more kinds of liquid oils as the component (B) can be used, and the content thereof in the total composition is preferably 3 to 23.75% by mass, or more preferably 4 to 20% by mass, in terms of excellent moisture retention in application and impact resistance.

An oil which is solid at 25 °C (solid oil) as the component (C) reversibly changes between solid and liquid states and is a hydrophobic compound having a melting point of 40°C or higher. The oil contains a wax formed of a hydrocarbon, ester, or silicone, and the wax is selected from, for example, an animal wax, a plant wax, a mineral wax, a synthetic wax, and a mixture thereof. Examples of the wax may include: animal waxes such as beeswax and whale wax; plant waxes such as carnauba wax, candelilla wax, rice wax, and Japan wax; mineral waxes such as montan wax, ozokerite, ceresin, paraffin wax, petrolatum, and microcrystalline wax; and synthetic waxes such as polyethylene wax, Fisher-Tropsch wax, hardened castor oil, hydrogenated jojoba oil, 12-hydroxystearicacid, amide stearate, imide phthalic anhydride, and silicone wax.

Of these waxes, ones having a penetration number of 15 or less when measured at 25°C by the method described in ASTM D-1321 are preferred in terms of glossiness on the surface of the molded cosmetic and impact resistance, or more preferred are waxes each having a penetration number of 10 or less. As oils which are solid at 25°C, waxes each having a penetration number of 15 or less and those other than the waxes can be used in combination. In the case of combined use, the total of oils which are solid at 25°C preferably has a penetration number of 15 or less at 25°C. Solid oils each having a penetration number of 15 or less are, for example, polyethylene wax, paraffin wax, candelilla wax, carnauba wax, rice wax, montan wax, ozokerite, ceresin, polyethylene wax, Fisher-Tropsch wax, hardened castor oil, hydrogenated jojoba oil, 12-hydroxystearic acid, amide stearate, imide phthalic anhydride, and silicone wax.

Further, the solid oils as the component (C) each having a higher melting point are preferred in terms of excellent impact resistance. Specifically, solid oils each having a melting point of 60°C or higher is preferred, or those each having a melting point of 70°C or higher are more preferred, and those each having a melting point of 110°C or lower are preferred in terms of excellent meltability.

In conventional molding using a high-pressure press, when a wax having a lower penetration number and a wax having a higher melting point were used, the degree of bonding between powders was not sufficient. However, in the present invention, it has been found that application of vibration in molding significantly strengthens bonding between powders.

One or more kinds of solid oils as the component (C) can be used, and the content thereof in the total composition is preferably 0.25 to 10% by mass, or more preferably 1 to 5% by mass in terms of excellent moisture retention in application and impact resistance.

Further, in the present invention, the mass ratio of the component (B) to the component (C) is 6:4 to 9.5:0.5, or preferably 7:3 to 9:1. When the mass ratio of the component (B) is larger, glossiness on the surface of the molded cosmetic is excellent, but impact resistance is inferior. When the mass ratio of the component (B) is smaller, moisture retention in application is not sufficiently provided.

In addition to the aforementioned components, the cosmetic of the present invention may appropriately contain components that are used for common cosmetics such as surfactants, antiseptics, antioxidants, pigments, perfumes, ultraviolet absorbers, moisturizers, bactericides, extinguishing agents, and skin activators.

In the present invention, a cosmetic can be produced, for example, by mixing a liquid oil (B) and a solid oil (C) under heating, further adding a powder (A) to the resultant to make a mixture (powder composition), and applying a vibration having a frequency of 10 to 40 kHz and an amplitude of 10 to 100 µm to the mixture, followed by molding under pressure. Further, preferred is producing a cosmetic by mixing uniformly the liquid oil (B) and the solid oil (C) with the powder (A) at a temperature at which the liquid oil (B) and the solid oil (C) melt.

To be more specific, a preferred method includes melting the liquid oil (B) and the solid oil (C) under heating to prepare an oily component, adding the oily component to the powder (A) preliminarily mixed at the melting temperature, and mixing the whole components uniformly. Spraying can also be adopted as a method of adding the oily component. The obtained mixture can be compacted without any further treatment. However, it is preferred that the mixture is cooled to a room temperature, and then the mixture be milled, followed by molding under pressure. The milled mixture of the components (A), (B), and (C) is passed through a sieve with a mesh size of 3 mm or less so that those passing through the sieve may be collected in a predetermined container for molding. Vibration is preferably applied to the mixture of the components (A), (B), and (C) in a state where about 10 g or more of weights or preferably 100 g or more of weights are applied.
The time of vibration application is preferably 0.1 to 2 seconds, or more preferably 0.5 to 1.5 seconds. After applying vibration, the mixture is retained with pressure being applied for preferably 0.1 second or longer. The mixture is retained for more preferably 0.1 to 5 seconds, or even more preferably 0.5 to 2 seconds, in terms of excellent impact resistance.

The pressure applied at the time of molding under pressure is preferably 10 kg/cm² or less, or more preferably 1 to 5 kg/cm² in terms of attachment to an applicator in use, attachment to the skin inuse, and moisture retention inapplication, and moreover, in terms of excellence in glossiness on the surface of the formed cosmetic and glossiness of the applied film.
By using high-pressure-type press molding that has been conventionally used, powders each having a larger particle size and each having a larger aspect ratio were crushed after the molding and changed to powders each having a smaller particle size. However, in the present invention, pressure at the time of molding under pressure is retained low, and hence the particle size distributions of powders before and after molding are not different, and further, the particle size distributions of the component (A) before and after molding are not different. Thus, provided is a cosmetic which is very high in glossiness on the surface of the molded cosmetic and in glossiness of the applied film.

The powder cosmetic to be obtained by the present invention is preferably used, for example, as make-up cosmetics such as a foundation, a face powder, a white powder, an eye shadow, an eyebrow cosmetic, and a blusher, or as body powders.

### [EXAMPLES]

Evaluation methods performed in examples are as described below.

### (Evaluation Methods)

(1) Measurement of Penetration number:
   An oil which was solid at 25°C was melted with heat and then cooled. The obtained product was used as a sample. The Penetration number of the sample was measured using a TESTING APPARATUS FOR

### PENETORATION (NIKKA ENGINEERING).

In other words, the sample was heated to a temperature that was 17°C higher than the melting point thereof so that it might be melted. Two cork stoppers (No. 16) were placed in line on a horizontal stand, and a brass plate was placed thereon. On the top face thereof, a parting agent (which was obtained by mixing glycerin andwateratequivalentamounts) was thinly applied. Apredetermined sample vessel (a brass cylinder with an inside diameter of 25.4 mm, a height of 31.8 mm, and a wall thickness of 3.2 mm) was placed on the brass plate. After that, a melted sample was poured into the sample vessel to such an extent that it could be seen from the superior margin of the vessel in a mound shape, and it was stood to cool at room temperature (22 to 26°C) for 1 hour. After that, the vessel was removed from the brass plate and then left in water with a constant temperature of 25°C for 1 hour. A predetermined needle having a total mass of 100 was caused to vertically penetrate into the sample for 5 seconds, and the dial gauge was read at the time so that the depth of the penetrated needle might be measured. The Penetration number of the sample was indicated with a value, which was obtained by measuring the depth of the penetrated needle at a unit of 0.1 mm and then increasing the value 10 times. Four measurement values were averaged, and the first decimal point of the average value was rounded. The resulting value was defined as a Penetration number.

(2) Impact Resistance:
   A powder cosmetic that had been filled in a middle plate with a height of 14 mm and a width of 26 mm followed by molding under pressure was placed in a vessel. After that, from a height of 30 cm, the vessel was repeatedly dropped onto an SUS board with a thickness of 25 mm. Based on the number of drops that was necessary for the occurrence of abnormality such as a chip or crack, impact resistance was evaluated in accordance with the following standards.
      A; 15 times or more
      B: 10 to 14 times
      C: 5 to 9 times
      D: 1 to 4 times

(3) Quality in Use:
   Ten special panelists used various powder cosmetics to conduct a sensory evaluation of the powder cosmetics in terms of uniformity in powder attachment to an applicator, uniformity in attachment to the skin, moisture retention in application, smoothness, glossiness and transparency of an applied film and glossiness on the surface of the formed cosmetic. Those factors were evaluated in accordance with the following standards.
      A; 7 or more panelists evaluated the cosmetic positively.
      B: 4 to 6 panelists evaluated the cosmetic positively.
      C: 2 or 3 panelists evaluated the cosmetic positively.
      D: 1 panelist or none evaluated the cosmetic positively.

### Examples 1 to 28 and Comparative Examples 1 to 6

Powder cosmetic with the compositions shown in Table 1 to Table 4 were produced. The produced cosmetics were evaluated in terms of impact resistance, uniformity in powder attachment to an applicator, uniformity in attachment to the skin, moisture retention in application, and glossiness on the surface of the molded cosmetic. The results are collectively shown in Table 1 to Table 4.

### (Production Method)

Powdery components were mixed and then heated to 80 to 90°C. Separately, a mixture of a liquid oil and a solid oil was heated to 80 to 90 °C so as to melt. The thus melted product was then added to the above mixture of powdery components and was uniformly blended. The resulting mixture was cooled and milled. Then, the resultant product was filled in a middle plate and subjected to molding under pressure while being applied with vibration under the conditions shown in Table 1 to Table 4. Thus, a powder cosmetic was obtained.

**[Table 1]**

| Component (% by mass) | | Examples | | | | | | | | | |
|---|---|---|---|---|---|---|---|---|---|---|---|
| | | 1 | 2 | 3 | 4 | 5 | 6 | 7 | 8 | 9 | 10 |
| A | Mica (average particle size of 20 µm) | 13 | 11 | 8 | 23 | 43 | 43 | 42 | 43 | 43 | 43 |
| | Sericite (average particle size of 8 µm) | 43 | 30 | 10 | 30 | 43 | 43 | 43 | 43 | 43 | 43 |
| | Pearl pigment (average particle size of 20 µm) | 30 | 40 | 50 | 10 | | | | | | |
| | Spherical silica (average particle size of 5 µm) | 2 | 2 | 5 | 2 | 2 | 2 | 2 | 2 | 2 | 2 |
| | Talc (average particle size of 7 µm) | | | | 28 | | | | | | |
| | Coloring pigment (average particle size of 0.1 µm) | 2 | 2 | 2 | 2 | 2 | 2 | 2 | 2 | 2 | 2 |
| B | Liquid isoparaffin | 8 | 12 | 20 | 4 | 7 | 6 | 9 | 9.5 | 8 | 8 |
| | Dimethylpolysiloxane | | | | | | | | | | |
| | Diisostearyl malate | | | | | | | | | | |
| C | Polyethylene wax (melting point of 100°C, penetration number of 1) | 2 | 3 | 5 | 1 | 3 | 4 | 1 | 0.5 | | |
| | Paraffin wax (melting point of 74°C; penetration number of 7) | | | | | | | | | 2 | |
| | Paraffin wax (melting point of 58°C; penetration number of 13) | | | | | | | | | | 2 |
| | Microcrystal line wax (melting point of 73°C; penetrationnumber of 34) | | | | | | | | | | |
| | Beeswax (melting point of 64°C; penetration number of 20) | | | | | | | | | | |
| | Carnauba wax (melting point of 83°C; penetration number of 1) | | | | | | | | | | |
| | Candelilla wax (melting point of 70°C; penetration number of 2) | | | | | | | | | | |
| | Content of component (A) (% by mass) | 90 | 85 | 75 | 95 | 90 | 90 | 90 | 90 | 90 | 90 |
| | (B) : (C) (Mass ratio) | 8:2 | 8:2 | 8:2 | 8:2 | 7:3 | 6:4 | 9:1 | 9.5: | 8:2 | 8:2 |
| Molding condition | Frequency (kHz) | 20 | 20 | 20 | 20 | 20 | 20 | 20 | 0.5 20 | 20 | 20 |
| | Amplitude (µm) | 20 | 20 | 20 | 20 | 20 | 20 | 20 | 20 | 20 | 20 |
| | Time of vibration application (second) | 1 | 1 | 1 | 1 | 1 | 1 | 0.75 | 0.75 | 1 | 1 |
| | Time of retention after vibration application (second) | 0.5 | 0.5 | 0.5 | 0.5 | 0.5 | 0.5 | 0.5 | 0.5 | 0.5 | 0.5 |
| | Press molding pressure (kg/cm²) | 4.2 | 4.2 | 2.8 | 4.2 | 2.8 | 4.2 | 4.2 | 4.2 | 4.2 | 4.2 |
| Evaluation | Impact resistance | A | A | A | A | A | A | A | B | A | B |
| | Uniformity in powder removal from applicator | A | A | A | A | A | A | A | A | A | A |
| | Uniformity in attachment to skin | A | A | A | A | A | B | A | A | A | A |
| | Moisture retention | A | A | A | A | A | B | A | A | A | A |
| | Glossiness on surface of molded cosmetic | A | A | A | A | A | A | A | B | A | A |

**[Table 2]**

| Component (% by mass) | | Examples | | | | | | Comparative Examples | | | |
|---|---|---|---|---|---|---|---|---|---|---|---|
| | | 11 | 12 | 13 | 14 | 15 | 16 | 1 | 2 | 3 | 4 |
| A | Mica (average particle size of 20 µm) | 43 | 43 | 43 | 43 | 43 | 43 | 43 | 43 | 43 | 43 |
| | Sericite (average particle size of 8 µm) | 43 | 43 | 43 | 43 | 43 | 43 | 43 | 43 | 43 | 43 |
| | Pearl pigment (average particle size of 20 µm) | | | | | | | | | | |
| | Spherical silica (average particle size of 5 µm) | 2 | 2 | 2 | 2 | 2 | 2 | 2 | 2 | 2 | 2 |
| | Talc (average particle size of 7 µm) | | | | | | | | | | |
| | Coloring pigment (average particle size of 0.1 µm) | 2 | 2 | 2 | 2 | 2 | 2 | 2 | 2 | 2 | 2 |
| B | Liquid isoparaffin | 8 | 8 | 8 | 8 | | | 8 | 8 | 5.5 | 9.8 |
| | Dimethylpolysiloxane | | | | | 8 | | | | | |
| | Diisostearyl malate | | | | | | 8 | | | | |
| C | Polyethylene wax (melting point of 100°C, penetration number of 1) | | | | | 2 | 2 | 2 | 2 | 4.5 | 0.2 |
| | Paraffin wax (melting point of 74°C; penetration number of 7) | | | | | | | | | | |
| | Paraffin wax (melting point of 58°C; penetration number of 13) | | | | | | | | | | |
| | Microcrystallinewax (melting point of 73°C; penetration number of 34) | 2 | | | | | | | | | |
| | Beeswax (melting point of 64°C; penetration number of 20) | | 2 | | | | | | | | |
| | Carnauba wax (melting point of 83°C; penetration number of 1) | | | 2 | | | | | | | |
| | Candelilla wax (melting point of 70°C; penetration number of 2) | | | | 2 | | | | | | |
| | Content of component (A) (% by mass) | 90 | 90 | 90 | 90 | 90 | 90 | 90 | 90 | 90 | 90 |
| | (B) : (C) (Mass ratio) | 8:2 | 8:2 | 8:2 | 8:2 | 8:2 | 8:2 | 8:2 | 8:2 | 5.5: 4.5 | 9.8: 0.2 |
| Molding condition | Frequency (kHz) | 20 | 20 | 20 | 20 | 20 | 20 | 20 | 20 | 20 | 20 |
| | Amplitude (µm) | 20 | 20 | 20 | 20 | 20 | 20 | 20 | 20 | 20 | 20 |
| | Time of vibration application (second) | 1 | 1 | 1 | 1 | 1 | 1 | 0 | 0 | 0.75 | 0.75 |
| | Time of retention after vibration application (second) | 0.5 | 0.5 | 0.5 | 0.5 | 0.5 | 0.5 | 0.5^{*1} | 1^{*1} | 0.5 | 0.5 |
| | Press molding pressure (kg/cm²) | 4.2 | 4.2 | 4.2 | 4.2 | 4.2 | 4.2 | 4.2 | 170 | 2.8 | 7.1 |
| Evaluation | Impact resistance | C | C | A | A | A | A | B | C | A | D |
| | Uniformity in powder removal from applicator | A | A | A | A | A | A | D | C | B | C |
| | Uniformity in attachment to skin | A | A | A | A | A | B | C | B | C | A |
| | Moisture retention | A | A | A | A | A | B | A | A | D | A |
| | Glossiness on surface of molded cosmetic | A | A | A | A | A | A | D | D | A | D |

| | | | | | | | | | | | |
|---|---|---|---|---|---|---|---|---|---|---|---|
| 1: Time of pressure application without vibration application | | | | | | | | | | | |

**[Table 3]**

| Component (% by mass) | | Examples | | | | | | | |
|---|---|---|---|---|---|---|---|---|---|
| | | 17 | 18 | 19 | 20 | 21 | 22 | 23 | 24 |
| A | Synthetic mica (average particle size of 40 µm, aspect ratio of 80)^{*1} | 20 | 30 | 20 | 20 | | | | |
| | Synthetic mica (average particle size of 5 µm, aspect ratio of 40)^{*2} | 10 | 40 | 10 | 10 | 20 | 68 | 60 | 87 |
| | Synthetic mica (average particle size of 10 µm, aspect ratio of 20)^{*3} | 20 | 10 | 20 | 20 | 60 | | | |
| | Mica (average particle size of 18 µm, aspect ratio of 60)^{*4} | | | | | | | | |
| | Sericite (average particle size of 7 µm, aspect ratio of 40)^{*5} | | | | | | | | |
| | Mica titanium (average particle size of 20 µm, aspect ratio of 40)^{*5} | | | | | | | | |
| | Glass powder coated with titanium oxide (average particle size of 40 µm, aspect ratio of 40)^{*7} | 20 | | 20 | 20 | | | | |
| | Glass powder coated with titanium oxide (average particle size of 80 µm, aspect ratio of 80)^{*8} | 1.0 | | 70 | 10 | | | | |
| | Talc (average particle size of 7 µm, aspect ratio of 15)^{*9} | 7 | 7 | 7 | 7 | 7 | 19 | 27 | |
| | Coloring pigment | 3 | 3 | 3 | 3 | 3 | 3 | 3 | 3 |
| C | Paraffin wax | 2 | 2 | 1 | 3 | 2 | 2 | 2 | 2 |
| B | Liquid isoparaffin | 8 | 8 | 9 | 7 | 8 | 8 | 8 | 8 |
| | Ratio of plate-like powders (aspect ratio of 20 to 400) in all powders (% by mass) | 88.9 | 88.9 | 88.9 | 88.9 | 88.9 | 75.6 | 66.7 | 96.7 |
| | (B):(C) (Mass ratio) | 8:2 | 8:2 | 9:1 | 7:3 | 8:2 | 8:2 | 8:2 | 8:2 |
| Molding condition | Frequency (kHz) | 20 | 20 | 20 | 20 | 20 | 20 | 20 | 20 |
| | Amplitude (µm) | 20 | 20 | 20 | 20 | 20 | 20 | 20 | 20 |
| | Time of irradiation (second) | 1 | 1 | 1 | 1 | 1 | 0.75 | 0.75 | 1 |
| | Time of retention (second) | 0.5 | 0.5 | 0.5 | 0.5 | 0.5 | 0.5 | 0.5 | 0.5 |
| | Press molding pressure (kg/cm²) | 4.2 | 4.2 | 4.2 | 4.2 | 4.2 | 4.2 | 2.8 | 4.2 |
| Evaluation | Impact resistance | A | A | B | A | A | A | A | B |
| | Uniformity in powder removal from applicator | A | A | B | A | A | A | A | B |
| | Uniformity in attachment to skin | A | A | B | A | A | A | A | B |
| | Moisture retention | A | A | A | B | B | C | C | B |
| | Smoothness | A | A | A | B | B | B | C | B |
| | Glossiness and transparency on surface of molded cosmetic | A | A | A | A | B | B | C | A |
| | Glossiness and transparency of applied film | A | A | A | A | B | B | C | A |

| | | | | | | | | | |
|---|---|---|---|---|---|---|---|---|---|
| *1: PDM-40L (Topy Industries Ltd.), *2: PDM-5L (Topy Industries Ltd.), *3: PDM-1000 (Topy Industries Ltd.) *4: Y-2300 (Yamaguchi Mica Co., Ltd.), *5: FSE (Sanshin Mining Ind. Co., Ltd.), *6: Timiron Starluster MP-115 (Merck & Co., Inc.) *7: Metashine 1040RS-S1 (Nippon Sheet Glass Co., Ltd.), *8: Metashine 1080RS-S1 (Nippon Sheet Glass Co., Ltd.), *9: JA-46R (Asada Milling Co., Ltd.) | | | | | | | | | |

**[Table 4]**

| Component (% by mass) | | Examples | | | | Comparative Examples | |
|---|---|---|---|---|---|---|---|
| | | 25 | 26 | 27 | 28 | 5 | 6 |
| A | Synthetic mica (average particle size of 40 µm, aspect ratio of 80)^{*1} | | | | | | |
| | Synthetic mica (average particle size of 5 µm, aspect ratio of 40)^{*2} | | | | | | |
| | Synthetic mica (average particle size of 10 µm, aspect ratio of 20)^{*3} | | | | 55 | | 55 |
| | Mica (average particle size of 18 µm, aspect ratio of 60)^{*4} | 70 | | | | 70 | |
| | Sericite (average particle size of 7 µm, aspect ratio of 40)^{*5} | | 70 | | | | |
| | Mica titanium (average particle size of 20 µm, aspect ratio of 40)^{*6} | | | 70 | | | |
| | Glass powder coated with titanium oxide (average particle size of 40 µm, aspect ratio of 40)^{*7} | | | | | | |
| | Glass powder coated with titanium oxide (average particle size of 80 µm, aspect ratio of 80)^{*8} | | | | 5 | | 5 |
| | Talc (average particle size of 7 µm, aspect ratio of 15)^{*9} | 17 | 17 | 17 | 30 | 17 | 30 |
| | Coloring pigment | 3 | 3 | 3 | | 3 | |
| C | Parraffin wax | 2 | 2 | 2 | 2 | 2 | 2 |
| B | Liquid isoparaffin | 8 | 8 | 8 | 8 | 8 | 8 |
| | Ratio of plate-like powders (aspect ratio of 20 to 400) in all powders (% by mass) | 77.8 | 77.8 | 77.8 | 66.7 | 77.8 | 66.7 |
| | (B) : (C) (Mass ratio) | 8:2 | 8:2 | 8:2 | 8:2 | 8:2 | 8:2 |
| Molding condition | Frequency (kHz) | 20 | 20 | 20 | 20 | - | - |
| | Amplitude (µm) | 20 | 20 | 20 | 20 | - | |
| | application (second) Time of vibration application (second) | 1 | 1 | 1 | 1 | - | - |
| | Time of retention after vibration application (second) | 0.5 | 0.5 | 0.5 | 0.5 | 0.5 | 0.5 |
| | Press molding pressure (kg/cm²) | 4.2 | 4.2 | 4.2 | 4.2 | 170 | 170 |
| Evaluation | Impact resistance | A | A | A | A | D | B |
| | Uniformity in powder removal from applicator | A | A | A | A | D | B |
| | Uniformity in attachment to skin | A | A | A | A | D | B |
| | Moisture retention | A | A | B | A | A | A |
| | Smoothness | A | A | B | A | A | A |
| | Glossiness and transparency on surface of molded cosmetic | A | A | A | A | D | D |
| | Glossiness and transparency of applied film | A | A | A | A | D | D |

### Example 29 (Foundation)

A foundation with the following composition was produced.

### (Production Method)

Components (1) to (10) were mixed and heated to 80 to 90°C. Components (11) and (12) were heated to 80 to 90°C so as to melt and mix with each other. The resulting mixture of components (11) and (12) was added to the above mixture of components (1) to (10) and was uniformly blended. The resulting mixture was cooled and then milled. After that, the resultant product was filled in a middle plate, followed by molding under pressure. Thus, a foundation was obtained. The molding under pressure was performed under the conditions of a frequency of 20 kHz, an amplitude of 20 µm, an irradiation time of 1 second, a retention time of 0.1 second, and a press molding pressure of 4.2 kg/cm².

| (Component) | (% by mass) |
|---|---|
| (1) Talc treated with silicone (average particle size: 7 µm) | 11.5 |
| (2) Mica treated with silicone (average particle size: 20 µm) | 35 |
| (3) Sericite treated with silicone (average particle size: 8 µm) | 20 |
| (4) Spherical silicone resin (average particle size: 5 µm) | 5 |
| (6) Yellow iron oxide treated with silicone (average particle size: 0.1 µm) | 0.8 |
| (7) Black iron oxide treated with silicone (average particle size: 0.1 µm) | 0.1 |
| (8) Red iron oxide treated with silicone (average particle size: 0.1 µm) | 2.5 |
| (9) Pearl pigment treated with silicone (average particle size: 10 µm) | 5 |
| (10) Antiseptic | 0.1 |
| (11) Liquid isoparaffin | 8 |
| (12) Polyethylene wax (penetration number: 1) | 2 |

### Example 30 (Eye Shadow)

An eye shadow with the following composition was produced.

### (Production Method)

Components (1) to (11) were mixed and heated to 80 to 90°C. Components (12) and (13) were heated to 80 to 90°C so as to melt and mix with each other. The resulting mixture of components (12) and (13) was added to the above mixture of components (1) to (11) and was uniformly blended. The resulting mixture was cooled and then milled. After that, the resultant product was filled in a middle plate, followed by molding under pressure. Thus, an eye shadow was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with silicone (average particle size: 7 µm) | 24.15 |
| (2) Synthetic mica treated with silicone (average particle size: 10 µm) | 30 |
| (3) Spherical nylon powder (average particle size: 5 µm) | 2 |
| (4) Titanium oxide treated with silicone (average particle size: 0.1 µm) | 0.1 |
| (5) Black iron oxide treated with silicone (average particle size: 0.1 µm) | 0.05 |
| (6) Ultramarine blue treated with silicone (average particle size: 0.1 µm) | 0.3 |
| (7) Red color 226 (average particle size: 0.1 µm) | 0.3 |
| (8) Mica titanium treated with silicone (average particle size: 20 µm) | 20 |
| (9) Mica titanium coated with red iron oxide treated with silicone (average particle size: 20 µm) | 8 |
| (10) Glass flake coated with titanium oxide treated with silicone (average particle size: 80 µm) | 5 |
| (11) Antiseptic | 0.1 |
| (12) Liquid isoparaffin | 8 |
| (13) Polyethylene wax (penetration number: 1) | 2 |

### Example 31 (White Powder)

A white powder with the following composition was produced.

### (Production Method)

Components (1) to (9) were mixed and heated to 80 to 90°C. Components (10) and (11) were heated to 80 to 90°C so as to melt and mix with each other. The resulting mixture of components (10) and (11) was added to the above mixture of components (1) to (9) and was uniformly blended. The resulting mixture was cooled and then milled. After that, the resultant product was filled in a middle plate, followed by molding under pressure. Thus, a white powder was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (weight %) |
|---|---|
| (1) Talc treated with fluorine compound (average particle size: 7 µm) | 34.19 |
| (2) Synthetic mica treated with fluorine compound (average particle size: 10 µm) | 50 |
| (3) Sericite treated with fluorine compound (average particle size: 8 µm) | 5 |
| (4) Spherical polymethyl methacrylate powder treated with fluorine compound (average particle size: 12 µm) | 2 |
| (5) Titanium oxide treated with fluorine compound (average particle size: 0.1 µm) | 0.5 |
| (6) Yellow iron oxide treated with fluorine compound (average particle size: 0.1 µm) | 0.1 |
| (7) Black iron oxide treated with fluorine compound (average particle size: 0.1 µm) | 0.01 |
| (8) Red iron oxide treated with fluorine compound (average particle size: 0.1 µm) | 0.1 |
| (9) Antiseptic | 0.1 |
| (10) Liquid isoparaffin | 6.4 |
| (11) Polyethylene wax (penetration number: 1) | 1.6 |

### Example 32 (Blusher)

A blusher with the following composition was produced.

### (Production Method)

Components (1) to (12) were mixed and heated to 80 to 90°C. Components (13) and (14) were heated to 80 to 90°C so as to melt and mix with each other. The resulting mixture of components (13) and (14) was added to the above mixture of component (1) to (12) and was uniformly blended. The resulting mixture was cooled and thenmilled. After that, he resultant product was filled in a middle plate, followed by molding under pressure. Thus, a blusher was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm) | 28.8 |
| (2) Mica treated with fluorine compound (average particle size: 10 µm) | 35 |
| (3) Sericite treated with fluorine compound (average particle size: 8 µm) | 8 |
| (4) Spherical silicone resin treated with fluorine compound (average particle size: 5 µm) | 2 |
| (5) Titanium oxide treated with fluorine compound (average particle size: 0.1 µm) | 0.5 |
| (6) Yellow iron oxide treated with fluorine compound (average particle size: 0.1 µm) | 0.3 |
| (7) Black iron oxide treated with fluorine compound (average particle size: 0.1 µm) | 0.1 |
| (8) Blue 404 treated with fluorine compound (average particle size: 0.1 µm) | 1.2 |
| (9) Mica titanium (average particle size: 20 µm) | 10 |
| (10) Mica titanium coated with red iron oxide (average particle size: 20 µm) | 2 |
| (11) Glass powder coated with titanium oxide (average particle size: 40 µm) | 4 |
| (12) Antiseptic | 0.1 |
| (13) Liquid isoparaffin | 6.4 |
| (14) Polyethylene wax (penetration number: 1) | 1.6 |

### Example 33 (Eyebrow cosmetic)

An eyebrow cosmetic with the following composition was produced.

### (Production method)

Components (1) to (9) were mixed and heated to 80 to 90°C. Components (10) and (11) were heated to 80 to 90°C so as to melt and mix with each other. The resulting mixture of components (10) and (11) was added to the above mixture of components (1) to (9) and was uniformly blended. The resulting mixture was cooled and then milled. After that, the resultant product was filled in a middle plate, followed by molding under pressure. Thus, an eyebrow cosmetic was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (averageparticle size: 7 µm) | 29.7 |
| (2) Mica treated with fluorine compound (averageparticle size: 10 µm) | 5 |
| (3) Sericite treated with fluorine compound (average particle size: 8 µm) | 40 |
| (4) Spherical nylon powder treated with fluorine compound (average particle size: 5 µm) | 5 |
| (5) Titanium oxide treated with fluorine compound (average particle size: 0.1 µm) | 0.5 |
| (6) Yellow iron oxide treated with fluorine compound (average particle size: 0.1 µm) | 0.2 |
| (7) Black iron oxide treated with fluorine compound (average particle size: 0.1 µm) | 12 |
| (8) Red iron treated with fluorine compound (average particle size: 0.1 µm) | 1.5 |
| (9) Antiseptic | 0.1 |
| (10) Liquid isoparaffin | 4.8 |
| (11) Polyethylene wax (penetration number: 1) | 1.2 |

### Example 34 (Body Powder)

A body powder with the following composition was produced.

### (Production Method)

Components (1) to (10) were mixed and heated to 80 to 90°C. Components (11) and (12) were heated to 80 to 90°C so as to melt and mix with each other. The resulting mixture of components (11) and (12) was added to the above mixture of components (1) to (10) and was uniformly blended. The resulting mixture was cooled and then milled. After that, the resultant product was filled in a middle plate, followed by molding under pressure. Thus, a body powder was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm) | 25.09 |
| (2) Mica treated with fluorine compound (average particle size: 10 µm) | 30 |
| (3) Sericite treated with fluorine compound (average particle size: 8 µm) | 30 |
| (4) Spherical silicone resin treated with fluorine compound (average particle size: 5 µm) | 6 |
| (5) Titanium oxide treated with fluorine compound (average particle size: 0.1 µm) | 0.5 |
| (6) Yellow iron oxide treated with fluorine compound (average particle size: 0.1 µm) | 0.1 |
| (7) Black iron oxide treated with fluorine compound (average particle size: 0.1 µm) | 0.01 |
| (8) Red iron oxide treated with fluorine compound (average particle size: 0.1 µm) | 0.1 |
| (9) Antiseptic | 0.1 |
| (10) Liquid isoparaffin | 6.4 |
| (11) Polyethylene wax (penetration number: 1) | 1.6 |
| (12) Antiphlogistic | 0.1 |

### Example 35 (Foundation)

A foundation with the following composition was produced.

### (Production Method)

Components (1) to (10) were mixed and heated to 80 to 90°C. Components (11) and (12) were heated to 80 to 90°C so as to melt and mix with each other. The resulting mixture of components (11) and (12) was added to the above mixture of components (1) to (10) and was uniformly blended. The resulting mixture was cooled and thenmilled. After that, the resultant product was filled in a middle plate, followed by molding under pressure. Thus, a foundation was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with silicone (average particle size:7 µm, aspect ratio: 15) | 9.5 |
| (2) Mica treated with silicone (average particle size:18 µm, aspect ratio: 60) | 40 |
| (3) Synthetic mica treated with silicone (average particle size: 20 µm, aspect ratio: 70) | 20 |
| (4) Glass powder treated with silicone (average particle size: 10 µm, aspect ratio: 25) | 5 |
| (5) Spherical silicone resin | 2 |
| (6) Titanium oxide treated with silicone | 10 |
| (7) Yellow iron oxide treated with silicone | 0.8 |
| (8) Black iron oxide treated with silicone | 0.1 |
| (9) Red iron oxide treated with silicone | 2.5 |
| (10) Antiseptic | 0.1 |
| (11) Liquid isoparaffin | 8 |
| (12) Polyethylene wax | 2 |

### Example 36 (Eye Shadow)

An eye shadow with the following composition was produced.

### (Production Method)

Components (1) to (11) were mixed and heated to 80 to 90°C. Components (12) and (13) were heated to 80 to 90°C so as to melt and mix with each other. The resulting mixture of components (12) and (13) was added to the above mixture of components (1) to (11) and was uniformly blended. The resulting mixture was cooled and then milled. After that, the resultant product was filled in a middle plate, followed by molding under pressure. Thus, an eye shadow was obtained. The molding under pressure was performed under the conditions of a frequency of 20 kHz, an amplitude of 20 µm, an irradiation time of 1 second, a retention time retention of 0.5 second, and a press molding pressure of 4.2 kg/cm².

| (Component) | (% by mass) |
|---|---|
| (1) Talc treated with silicone (average particle size: 7 µm, aspect ratio: 15) | 24.15 |
| (2) Synthetic mica treated with silicone (average particle size: 10 µm, aspect ratio: 60) | 25 |
| (3) Spherical nylon powder (average particle size:5 µm) | 2 |
| (4) Titanium oxide treated with silicone | 0.1 |
| (5) Black iron oxide treated with silicone | 0.05 |
| (6) Ultramarine blue treated with silicone | 0.3 |
| (7) Red color 226 | 0.3 |
| (8) Mica titanium treated with silicone (average particle size: 20 µm, aspect ratio: 40) | 20 |
| (9) Mica titanium coated with red iron oxide treated with silicone (average particle size: 20 µm, aspect ratio: 40) | 8 |
| (10) Glass powder coated with titanium oxide treatedwith silicone (average particle size: 80 µm, aspect ratio: 80) | 10 |
| (11) Antiseptic | 0.1 |
| (12) Liquid isoparaffin | 8 |
| (13) Polyethylene wax | 2 |

### Example 37 (Face Powder)

A face powder with the following composition was produced.

### (Production Method)

Components (1) to (10) were mixed and heated to So to 90°C. Components (11) and (12) were heated to 80 to 90°C so as to melt and mix with each other. The resulting mixture of components (11) and (12) was added to the above mixture of components (1) to (10) and was uniformly blended. The resulting mixture was cooled and then milled. After that, the resultant product was filled in a middle plate, followed by molding under pressure. Thus, a face powder was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm, aspect ratio: 15) | 24.19 |
| (2) Synthetic mica treated with fluorine compound (average particle size: 10 µm, aspect ratio: 60) | 50 |
| (3) Sericite treated with fluorine compound (average particle size: 8 µm, aspect ratio: 40) | 5 |
| (4) Glass powder (average particle size: 25 µm, aspec tratio: 60) | 10 |
| (5) Spherical polymethyl methacrylate powder treated with fluorine compound (average particle size: 12 µm) | 2 |
| (6) Titanium oxide treated with fluorine compound | 0.5 |
| (7) Yellow iron oxide treated with fluorine compound | 0.1 |
| (8) Black iron oxide treated with fluorine compound | 0.01 |
| (9) Red iron oxide treated with fluorine compound | 0.1 |
| (10) Antiseptic | 0.1 |
| (11) Liquid isoparaffin | 6.4 |
| (12) Polyethylene wax | 1.6 |

### Example 38 (Blusher)

A blusher with the following composition was produced.

### (Component)

Components (1) to (13) were mixed and heated to 80 to 90°C. Components (14) and (15) were heated to 80 to 90°C so as to melt and mix with each other. The resulting mixture of components (14) and (15) was added to the above mixture of components (1) to (13) and was uniformly blended. The resulting mixture was cooled and then milled. After that, the resultant product was filled in a middle plate, followed by molding under pressure. Thus, a blusher was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm, aspect ratio: 15) | 18.8 |
| (2) Synthetic mica treated with fluorine compound (average particle size: 10 µm, aspect ratio: 30) | 20 |
| (3) Sericite treated with fluorine compound (average particle size: 8 µm, aspect ratio: 40) | 13 |
| (4) Glass powder (average particle size: 25 µm,aspect ratio: 60) | 20 |
| (5) Spherical silicone resin treated with fluorine compound (average particle size: 5 µm) | 2 |
| (6) Titanium oxide treated with fluorine compound | 0.5 |
| (7) Yellow iron oxide treated with fluorine compound | 0.3 |
| (8) Black iron oxide treated with fluorine compound | 0.1 |
| (9) Blue 404 treated with fluorine compound | 1.2 |
| (10) Mica titanium (average particle size: 20 µm, aspect ratio: 40) | 10 |
| (11) Mica titanium coated with red iron oxide (average particle size: 20 µm, aspect ratio: 40) | 2 |
| (12) Glass powder coated with titanium oxide (average particle size: 40 µm, aspect ratio: 40) | 4 |
| (13) Antiseptic | 0.1 |
| (14) Liquid isoparaffin | 6.4 |
| (15) Polyethylene wax | 1.6 |

### Example 39 (Eyebrow cosmetic)

An eyebrow cosmetic with the following composition was produced.

### (Production method)

Components (1) to (11) were mixed and heated to 80 to 90°C. Components (12) and (13) were heated to 80 to 90°C so as to melt and mix with each other. The resulting mixture of components (12) and (13) was added to the above mixture of components and was uniformly blended. The resulting mixture was cooled and then milled. After that, the resultant product was filled in a middle plate, followed by molding under pressure. Thus, an eyebrow cosmetic was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm, aspect ratio: 15) | 2.7 |
| (2) Synthetic mica treated with fluorine compound (average particle size: 10 µm, aspect ratio: 30) | 22 |
| (3) Sericite treated with fluorine compound (average particle size: 8 µm, aspect ratio: 40) | 20 |
| (4) Glass powder (average particle size: 10 µm,aspect ratio: 25) | 10 |
| (5) Mica titanium coated with red iron oxide (average particle size: 20 µm, aspect ratio: 40) | 20 |
| (6) Spherical nylon powder treated with fluorine compound (average particle size: µm) | 5 |
| (7) Titanium oxide treated with fluorine compound | 0.5 |
| (8) Yellow iron oxide treated with fluorine compound | 0.2 |
| (9) Black iron oxide treated with fluorine compound | 12 |
| (10) Red iron oxide treated with fluorine compound | 1.5 |
| (11) Antiseptic | 0.1 |
| (12) Liquid isoparaffin | 4.8 |
| (13) Polyethylene wax | 1.2 |

### Example 40 (Body Powder)

A body powder with the following composition was produced.

### (Production Method)

Components (1) to (11) were mixed and heated to 80 to 90°C. Components (12) to (14) were heated to 80 to 90°C so as to melt and mix with each other. The resulting mixture of components (12) to (14) was added to the above mixture of components (1) to (11) and was uniformly blended. The resulting mixture was cooled and then milled. After that, the resultant product was filled in a middle plate, followed by molding under pressure. Thus, a body powder was obtained. The molding under pressure was performed under the conditions of a frequency of 20 kHz, an amplitude of 20 µm, an irradiation time of 1 second, a retention time of 0.5 second, and a press molding pressure of 4.2 kg/cm².

| (Component) | (% by mass) |
|---|---|
| (1) Talc treated with fluorine compound (average particle size: 7 µm, aspect ratio: 15) | 15.09 |
| (2) Synthetic mica treated with fluorine compound (average particle size: 10 µm, aspect ratio: 30) | 10 |
| (3) Sericite treated with fluorine compound (average particle size: 8 µm, aspect ratio: 40) | 30 |
| (4) Glass powder (average particle size: 25 µm,aspect ratio: 60) | 20 |
| (5) Mica titanium (average particle size: 20 µm,aspect ratio: 40) | 10 |
| (6) Spherical silicone resin treated with fluorine compound (average particle size: 5 µm) | 6 |
| (7) Titanium oxide treated with fluorine compound | 0.5 |
| (8) Yellow iron oxide treated with fluorine compound | 0.1 |
| (9) Black iron oxide treated with fluorine compound | 0.01 |
| (10) Red iron oxide treated with fluorine compound | 0.1 |
| (11) Antiseptic | 0.1 |
| (12) Liquid isoparaffin | 6.4 |
| (13) Polyethylene wax | 1.6 |
| (14) Antiphlogistic | 0.1 |

All the powder cosmetics obtained in Examples 29 to 40 were excellent in terms of impact resistance, uniformity in attachment to an applicator, uniformity in attachment to the skin, moisture retention in application, and glossiness and transparency on the surface of the molded cosmetic.

### Test Example 1

The powder cosmetics obtained in Example 28 and Comparative Example 6 were measured for the particle size distribution of the total powder components contained in each of the cosmetics by dispersing powders in ethanol using a laser diffraction particle size analyzer (A-920, HORIBA, Ltd.). FIG. 1 illustrates the results.
The results in FIG. 1 show that the powder cosmetic of the present invention contains powders each having a particle size of about 100 µm because the powders were not crushed owing to irradiation with an ultrasonic wave at the time of molding under pressure even if the powder cosmetic of the present invention has the same composition as that of Comparative Example 6. Thus, a powder cosmetic excellent in glossiness on the surface of the molded cosmetics is provided.

## Claims

1. A method of producing a cosmetic by molding a powder composition under pressure, the method comprising applying a vibration having a frequency of 10 to 40 kHz and an amplitude of 10 to 100 µm to the powder composition while the powder composition is molded under pressure, the powder composition containing:
(A) a powder having an average particle size of 0.1 to 300 µm;
(B) an oil which is liquid at 25°C; and
(C) an oil which is solid at 25°C, wherein:
a content of the component (A) is 75 to 95% by mass; and
a mass ratio of the component (B) to the component (C) " (B) : (C) is 6:4 to 9.5:0.5.

2. The method of producing a cosmetic according to claim 1, wherein the pressure applied during the molding is 10 kg/cm² or less.

3. The method of producing a cosmetic according to claim 1 or 2, wherein the powder as the component (A) contains one or more kinds of plate-like powders selected from mica, synthetic mica, sericite, glass, and a pearl pigment at 30 to 95% by mass.

4. The method of producing a cosmetic according to any one of claims 1 to 3, wherein the component (C) has a penetration number of 15 or less.

5. The method of producing a cosmetic according to any one of claims 1 to 4, wherein the component (C) has a melting point of 60°C or lower.

6. A cosmetic obtained by the method of producing a cosmetic according to any one of claims 1 to 5.
